# EUROPEAN PATENT APPLICATION

(11) **EP 1 661 912 A1**
(43) Date of publication of application: **31.05.2006**
(21) Application number: 04028278.2
(22) Date of filing: 29.11.2004
(51) Int. Cl.: C07K 14/47, C12N 15/62, A61K 38/17, C12N 15/12

(54) **Fusion protein comprising a BH3-domain of a BH3-only protein**

(71) Applicant: Xigen S.A., 1005 Lausanne (CH)
(72) Inventor: Bonny, Christophe, 1006 lausanne (CH); Coquoz, Didier, 1025 St. Sulpice (CH)
(74) Representative: Graf von Stosch, Andreas

(57) **Abstract**

This invention relates to a fusion protein comprising at least one first portion (I) comprising a trafficking sequence and at least one second portion (II) comprising a full-length or partial BH3-domain sequence of a BH3-only protein, said fusion protein comprising D-enantiomeric amino acids in retro-inverso order in its portion (I). Furthermore, the invention relates to pharmaceutical compositions containing said fusion protein as well as to the use of said fusion protein.

SEQUENCE LISTING

<110> Xigen S.A.

<120> Fusion proteins comprising BH3 domains of BH3-only proteins of Bcl-2
family proteins

<130> CX01P002EP

<160> 14

<170> PatentIn version 3.1

<210> 1

<211> 10

<212> PRT

<213> Homo sapiens

<220>

<221> MISC_FEATURE

<223> Description of sequence: amino acid sequence of D-Tat

<220>

<221> MISC_FEATURE

<222> (10)..(10)

<223> Xaa = Gly or deleted

<220>

<221> MOD_RES

<222> (1)..(10)

<223> amino acids 1 to 10 are D-amino acids

<400>  1

Arg Arg Arg Gln Arg Arg Lys Lys Arg Xaa
1               5                   10


<210>  2

<211>  9

<212>  PRT

<213>  Homo sapiens



<220>

<221>  MISC_FEATURE

<222>  (1)..(9)

<223>  Xaa = arginine or lysine at positions 1-3 and 5 to 9, and
       Xaa = any non-basic amino acid at position 4



<220>

<221>  MOD_RES

<222>  (1)..(9)

<223>  amino acids 1 to 9 are D-amino acids



<400>  2

Xaa Xaa Xaa Xaa Xaa Xaa Xaa Xaa Xaa
1               5


<210>  3

<211>  241

<212>  PRT

<213>  Homo sapiens



<220>

<221>  MISC_FEATURE

<223>  Description of sequence: amino acid sequence of Bid (human) (tran
       script variant 1)



<400>  3

Met Cys Ser Gly Ala Gly Val Met Met Ala Arg Trp Ala Ala Arg Gly
1               5                   10                  15

Arg Ala Gly Trp Arg Ser Thr Val Arg Ile Leu Ser Pro Leu Gly His
            20                  25                  30

Cys Glu Pro Gly Val Ser Arg Ser Cys Arg Ala Ala Gln Ala Met Asp
        35                  40                  45

Cys Glu Val Asn Asn Gly Ser Ser Leu Arg Asp Glu Cys Ile Thr Asn
    50                  55                  60

Leu Leu Val Phe Gly Phe Leu Gln Ser Cys Ser Asp Asn Ser Phe Arg
65                  70                  75                  80

Arg Glu Leu Asp Ala Leu Gly His Glu Leu Pro Val Leu Ala Pro Gln
                85                  90                  95

Trp Glu Gly Tyr Asp Glu Leu Gln Thr Asp Gly Asn Arg Ser Ser His
            100                 105                 110

Ser Arg Leu Gly Arg Ile Glu Ala Asp Ser Glu Ser Gln Glu Asp Ile
        115                 120                 125

Ile Arg Asn Ile Ala Arg His Leu Ala Gln Val Gly Asp Ser Met Asp
    130                 135                 140

Arg Ser Ile Pro Pro Gly Leu Val Asn Gly Leu Ala Leu Gln Leu Arg
145                 150                 155                 160

Asn Thr Ser Arg Ser Glu Glu Asp Arg Asn Arg Asp Leu Ala Thr Ala
                165                 170                 175

Leu Glu Gln Leu Leu Gln Ala Tyr Pro Arg Asp Met Glu Lys Glu Lys
            180                 185                 190

Thr Met Leu Val Leu Ala Leu Leu Leu Ala Lys Lys Val Ala Ser His
        195                 200                 205

Thr Pro Ser Leu Leu Arg Asp Val Phe His Thr Thr Val Asn Phe Ile
    210                 215                 220

Asn Gln Asn Leu Arg Thr Tyr Val Arg Ser Leu Ala Arg Asn Gly Met
225                 230                 235                 240

Asp

<210>  4

<211>  168

<212>  PRT

<213>  Homo sapiens

<220>

<221>  MISC_FEATURE

<223>  Description of sequence: amino acid sequence of Bad (human)

<400>  4

Met Phe Gln Ile Pro Glu Phe Glu Pro Ser Glu Gln Glu Asp Ser Ser
1               5                   10                  15

Ser Ala Glu Arg Gly Leu Gly Pro Ser Pro Ala Gly Asp Gly Pro Ser
            20                  25                  30

Gly Ser Gly Lys His His Arg Gln Ala Pro Gly Leu Leu Trp Asp Ala
        35                  40                  45

Ser His Gln Gln Glu Gln Pro Thr Ser Ser Ser His His Gly Gly Ala
    50                  55                  60

Gly Ala Val Glu Ile Arg Ser Arg His Ser Ser Tyr Pro Ala Gly Thr
65                  70                  75                  80

Glu Asp Asp Glu Gly Met Gly Glu Glu Pro Ser Pro Phe Arg Gly Arg
                85                  90                  95

Ser Arg Ser Ala Pro Pro Asn Leu Trp Ala Ala Gln Arg Tyr Gly Arg
            100                 105                 110

Glu Leu Arg Arg Met Ser Asp Glu Phe Val Asp Ser Phe Lys Lys Gly
        115                 120                 125

Leu Pro Arg Pro Lys Ser Ala Gly Thr Ala Thr Gln Met Arg Gln Ser
    130                 135                 140

Ser Ser Trp Thr Arg Val Phe Gln Ser Trp Trp Asp Arg Asn Leu Gly
145                 150                 155                 160

Arg Gly Ser Ser Ala Pro Ser Gln

                165

<210>  5

<211>  483

<212>  PRT

<213>  Homo sapiens

<220>

<221>  misc_feature

<223>  Description of sequence: amino acid sequence of Noxa1 (human)

<400>  5

Met Ala Ser Leu Gly Asp Leu Val Arg Ala Trp His Leu Gly Ala Gln
1               5                   10                  15

Ala Val Asp Arg Gly Asp Trp Ala Arg Ala Leu His Leu Phe Ser Gly
            20                  25                  30

Val Pro Ala Pro Pro Ala Arg Leu Cys Phe Asn Ala Gly Cys Val His
        35                  40                  45

Leu Leu Ala Gly Asp Pro Glu Ala Ala Leu Arg Ala Phe Asp Gln Ala
    50                  55                  60

Val Thr Lys Asp Thr Cys Met Ala Val Gly Phe Phe Gln Arg Gly Val
65                  70                  75                  80

Ala Asn Phe Gln Leu Ala Arg Phe Gln Glu Ala Leu Ser Asp Phe Trp
                85                  90                  95

Leu Ala Leu Glu Gln Leu Arg Gly His Ala Ala Ile Asp Tyr Thr Gln
            100                 105                 110

Leu Gly Leu Arg Phe Lys Leu Gln Ala Trp Glu Val Leu His Asn Val
        115                 120                 125

Ala Ser Ala Gln Cys Gln Leu Gly Leu Trp Thr Glu Ala Ala Ser Ser
    130                 135                 140

Leu Arg Glu Ala Met Ser Lys Trp Pro Glu Gly Ser Leu Asn Gly Leu
145                 150                 155                 160

Asp Ser Ala Leu Asp Gln Val Gln Arg Arg Gly Ser Leu Pro Pro Arg
                165                 170                 175

Gln Val Pro Arg Gly Glu Val Phe Arg Pro His Arg Trp His Leu Lys
            180                 185                 190

His Leu Glu Pro Val Asp Phe Leu Gly Lys Ala Lys Val Val Ala Ser
        195                 200                 205

Ala Ile Pro Asp Asp Gln Gly Trp Gly Val Arg Pro Gln Gln Pro Gln
    210                 215                 220

Gly Pro Gly Ala Asn His Asp Ala Arg Ser Leu Ile Met Asp Ser Pro
225                 230                 235                 240

Arg Ala Gly Thr His Gln Gly Pro Leu Asp Ala Glu Thr Glu Val Gly
                245                 250                 255

Ala Asp Arg Cys Thr Ser Thr Ala Tyr Gln Glu Gln Arg Pro Gln Val
            260                 265                 270

Glu Gln Val Gly Lys Gln Ala Pro Leu Ser Pro Gly Leu Pro Ala Met
        275                 280                 285

Gly Gly Pro Gly Pro Gly Pro Cys Glu Asp Pro Ala Gly Ala Gly Gly
    290                 295                 300

Ala Gly Ala Gly Gly Ser Glu Pro Leu Val Thr Val Thr Val Gln Cys
305                 310                 315                 320

Ala Phe Thr Val Ala Leu Arg Ala Arg Arg Gly Ala Asp Leu Ser Ser
                325                 330                 335

Leu Arg Ala Leu Leu Gly Gln Ala Leu Pro His Gln Ala Gln Leu Gly
            340                 345                 350

Gln Leu Ser Tyr Leu Ala Pro Gly Glu Asp Gly His Trp Val Pro Ile
        355                 360                 365

Pro Glu Glu Glu Ser Leu Gln Arg Ala Trp Gln Asp Ala Ala Ala Cys
    370                 375                 380

Pro Arg Gly Leu Gln Leu Gln Cys Arg Gly Ala Gly Gly Arg Pro Val
385                 390                 395                 400

Leu Tyr Gln Val Val Ala Gln His Ser Tyr Ser Ala Gln Gly Pro Glu
                405                 410                 415

Asp Leu Gly Phe Arg Gln Gly Asp Thr Val Asp Val Leu Cys Glu Glu
            420                 425                 430

Pro Asp Val Pro Leu Ala Val Asp Gln Ala Trp Leu Glu Gly His Cys
        435                 440                 445

Asp Gly Arg Ile Gly Ile Phe Pro Lys Cys Phe Val Val Pro Ala Gly
    450                 455                 460

Pro Arg Met Ser Gly Ala Pro Gly Arg Leu Pro Arg Ser Gln Gln Gly
465                 470                 475                 480

Asp Gln Pro

<210>  6

<211>  193

<212>  PRT

<213>  Homo sapiens

<220>

<221>  MISC_FEATURE

<223>  Description of sequence: amino acid sequence of Puma (human)

<400>  6

Met Ala Arg Ala Arg Gln Glu Gly Ser Ser Pro Glu Pro Val Glu Gly
1               5                   10                  15

Leu Ala Arg Asp Gly Pro Arg Pro Phe Pro Leu Gly Arg Leu Val Pro
            20                  25                  30

Ser Ala Val Ser Cys Gly Leu Cys Glu Pro Gly Leu Ala Ala Ala Pro
        35                  40                  45

Ala Ala Pro Thr Leu Leu Pro Ala Ala Tyr Leu Cys Ala Pro Thr Ala
    50                  55                  60

Pro Pro Ala Val Thr Ala Ala Leu Gly Gly Ser Arg Trp Pro Gly Gly
65                  70                  75                  80

Pro Arg Ser Arg Pro Arg Gly Pro Arg Pro Asp Gly Pro Gln Pro Ser
                85                  90                  95

Leu Ser Leu Ala Glu Gln His Leu Glu Ser Pro Val Pro Ser Ala Pro
            100                 105                 110

Gly Ala Leu Ala Gly Gly Pro Thr Gln Ala Ala Pro Gly Val Arg Gly
        115                 120                 125

Glu Glu Glu Gln Trp Ala Arg Glu Ile Gly Ala Gln Leu Arg Arg Met
    130                 135                 140

Ala Asp Asp Leu Asn Ala Gln Tyr Glu Arg Arg Arg Gln Glu Glu Gln
145                 150                 155                 160

Gln Arg His Arg Pro Ser Pro Trp Arg Val Leu Tyr Asn Leu Ile Met
                165                 170                 175

Gly Leu Leu Pro Leu Pro Arg Gly His Arg Ala Pro Glu Met Glu Pro
            180                 185                 190

Asn

<210> 7

<211> 198

<212> PRT

<213> Homo sapiens

<220>

<221> MISC_FEATURE

<223> Description of sequence: amino acid sequence of Bim (human) (tran
script variant 1)

<400> 7

<210>  8

<211>  160

<212>  PRT

<213>  Homo sapiens

<220>

<221>  MISC_FEATURE

<223>  Description of sequence: amino acid sequence of Bik (human)

<400>  8

Met Ser Glu Val Arg Pro Leu Ser Arg Asp Ile Leu Met Glu Thr Leu
1               5                   10                  15

Leu Tyr Glu Gln Leu Leu Glu Pro Pro Thr Met Glu Val Leu Gly Met
            20                  25                  30

Thr Asp Ser Glu Glu Asp Leu Asp Pro Met Glu Asp Phe Asp Ser Leu
        35                  40                  45

Glu Cys Met Glu Gly Ser Asp Ala Leu Ala Leu Arg Leu Ala Cys Ile
    50                  55                  60

Gly Asp Glu Met Asp Val Ser Leu Arg Ala Pro Arg Leu Ala Gln Leu
65                  70                  75                  80

Ser Glu Val Ala Met His Ser Leu Gly Leu Ala Phe Ile Tyr Asp Gln
                85                  90                  95

Thr Glu Asp Ile Arg Asp Val Leu Arg Ser Phe Met Asp Gly Phe Thr
            100                 105                 110

Thr Leu Lys Glu Asn Ile Met Arg Phe Trp Arg Ser Pro Asn Pro Gly
        115                 120                 125

Ser Trp Val Ser Cys Glu Gln Val Leu Leu Ala Leu Leu Leu Leu Leu
    130                 135                 140

Ala Leu Leu Leu Pro Leu Leu Ser Gly Gly Leu His Leu Leu Leu Lys
145                 150                 155                 160

<210>  9

<211>  251

<212>  PRT

<213>  Homo sapiens

<220>

<221>  MISC_FEATURE

<223>  Description of sequence: amino acid sequence of D-Tat-Bid (human)
        (transcript variant 1)

<220>

<221>  MISC_FEATURE

<222>  (10)..(10)

<223>  Xaa = Gly or deleted

<220>

<221>  MOD_RES

<222>  (1)..(10)

<223>  amino acids 1 to 10 are D-amino acids

<400>  9

Arg Arg Arg Gln Arg Arg Lys Lys Arg Xaa Met Cys Ser Gly Ala Gly
1               5                   10                  15

Val Met Met Ala Arg Trp Ala Ala Arg Gly Arg Ala Gly Trp Arg Ser
            20                  25                  30

Thr Val Arg Ile Leu Ser Pro Leu Gly His Cys Glu Pro Gly Val Ser
        35                  40                  45

Arg Ser Cys Arg Ala Ala Gln Ala Met Asp Cys Glu Val Asn Asn Gly
    50                  55                  60

Ser Ser Leu Arg Asp Glu Cys Ile Thr Asn Leu Leu Val Phe Gly Phe
65                  70                  75                  80

Leu Gln Ser Cys Ser Asp Asn Ser Phe Arg Arg Glu Leu Asp Ala Leu
                85                  90                  95

Gly His Glu Leu Pro Val Leu Ala Pro Gln Trp Glu Gly Tyr Asp Glu
            100                 105                 110

Leu Gln Thr Asp Gly Asn Arg Ser Ser His Ser Arg Leu Gly Arg Ile
        115                 120                 125

Glu Ala Asp Ser Glu Ser Gln Glu Asp Ile Ile Arg Asn Ile Ala Arg
    130                 135                 140

His Leu Ala Gln Val Gly Asp Ser Met Asp Arg Ser Ile Pro Pro Gly
145                 150                 155                 160

Leu Val Asn Gly Leu Ala Leu Gln Leu Arg Asn Thr Ser Arg Ser Glu
                165                 170                 175

Glu Asp Arg Asn Arg Asp Leu Ala Thr Ala Leu Glu Gln Leu Leu Gln
            180                 185                 190

Ala Tyr Pro Arg Asp Met Glu Lys Glu Lys Thr Met Leu Val Leu Ala
        195                 200                 205

Leu Leu Leu Ala Lys Lys Val Ala Ser His Thr Pro Ser Leu Leu Arg
    210                 215                 220

Asp Val Phe His Thr Thr Val Asn Phe Ile Asn Gln Asn Leu Arg Thr
225                 230                 235                 240

Tyr Val Arg Ser Leu Ala Arg Asn Gly Met Asp
                245                 250

<210> 10

<211> 178

<212> PRT

<213> Homo sapiens

<220>

<221> MISC_FEATURE

<223> Description of sequence: amino acid sequence of D-Tat-Bad (human)

<220>

<221> MISC_FEATURE

<222> (10)..(10)

<223> Xaa = Gly or deleted

<220>

<221> MOD_RES

<222> (1)..(10)

<223> amino acids 1 to 10 are D-amino acids

<400> 10

Arg Arg Arg Gln Arg Arg Lys Lys Arg Gly Met Phe Gln Ile Pro Glu
1               5                   10                  15

Phe Glu Pro Ser Glu Gln Glu Asp Ser Ser Ser Ala Glu Arg Gly Leu
            20                  25                  30

Gly Pro Ser Pro Ala Gly Asp Gly Pro Ser Gly Ser Gly Lys His His
      35                  40                  45

Arg Gln Ala Pro Gly Leu Leu Trp Asp Ala Ser His Gln Gln Glu Gln
    50                  55                  60

Pro Thr Ser Ser Ser His His Gly Gly Ala Gly Ala Val Glu Ile Arg
65                  70                  75                  80

Ser Arg His Ser Ser Tyr Pro Ala Gly Thr Glu Asp Asp Glu Gly Met
                85                  90                  95

Gly Glu Glu Pro Ser Pro Phe Arg Gly Arg Ser Arg Ser Ala Pro Pro
            100                 105                 110

Asn Leu Trp Ala Ala Gln Arg Tyr Gly Arg Glu Leu Arg Arg Met Ser
      115                 120                 125

Asp Glu Phe Val Asp Ser Phe Lys Lys Gly Leu Pro Arg Pro Lys Ser
    130                 135                 140

Ala Gly Thr Ala Thr Gln Met Arg Gln Ser Ser Ser Trp Thr Arg Val
145                 150                 155                 160

Phe Gln Ser Trp Trp Asp Arg Asn Leu Gly Arg Gly Ser Ser Ala Pro
                165                 170                 175

Ser Gln

<210>  11

<211>  493

<212>  PRT

<213>  Homo sapiens

<220>

<221>  misc_feature

<223>  Description of sequence: amino acid sequence of D-Tat Noxa1 (huma
       n)

<220>

<221>  misc_feature

<222>  (10)..(10)

<223>  Xaa = Gly or deleted



<220>

<221>  MOD_RES

<222>  (1)..(10)

<223>  amino acids 1 to 10 are D-amino acids



<400>  11

Arg Arg Arg Gln Arg Arg Lys Lys Arg Gly Met Ala Ser Leu Gly Asp
1               5                   10                  15

Leu Val Arg Ala Trp His Leu Gly Ala Gln Ala Val Asp Arg Gly Asp
            20                  25                  30

Trp Ala Arg Ala Leu His Leu Phe Ser Gly Val Pro Ala Pro Pro Ala
        35                  40                  45

Arg Leu Cys Phe Asn Ala Gly Cys Val His Leu Leu Ala Gly Asp Pro
    50                  55                  60

Glu Ala Ala Leu Arg Ala Phe Asp Gln Ala Val Thr Lys Asp Thr Cys
65                  70                  75                  80

Met Ala Val Gly Phe Phe Gln Arg Gly Val Ala Asn Phe Gln Leu Ala
                85                  90                  95

Arg Phe Gln Glu Ala Leu Ser Asp Phe Trp Leu Ala Leu Glu Gln Leu
            100                 105                 110

Arg Gly His Ala Ala Ile Asp Tyr Thr Gln Leu Gly Leu Arg Phe Lys
        115                 120                 125

Leu Gln Ala Trp Glu Val Leu His Asn Val Ala Ser Ala Gln Cys Gln
    130                 135                 140

Leu Gly Leu Trp Thr Glu Ala Ala Ser Ser Leu Arg Glu Ala Met Ser
145                 150                 155                 160

Lys Trp Pro Glu Gly Ser Leu Asn Gly Leu Asp Ser Ala Leu Asp Gln
                165                 170                 175

Val Gln Arg Arg Gly Ser Leu Pro Pro Arg Gln Val Pro Arg Gly Glu
            180                 185                 190

Val Phe Arg Pro His Arg Trp His Leu Lys His Leu Glu Pro Val Asp
        195                 200                 205

Phe Leu Gly Lys Ala Lys Val Val Ala Ser Ala Ile Pro Asp Asp Gln
    210                 215                 220

Gly Trp Gly Val Arg Pro Gln Gln Pro Gln Gly Pro Gly Ala Asn His
225                 230                 235                 240

Asp Ala Arg Ser Leu Ile Met Asp Ser Pro Arg Ala Gly Thr His Gln
                245                 250                 255

Gly Pro Leu Asp Ala Glu Thr Glu Val Gly Ala Asp Arg Cys Thr Ser
            260                 265                 270

Thr Ala Tyr Gln Glu Gln Arg Pro Gln Val Glu Gln Val Gly Lys Gln
        275                 280                 285

Ala Pro Leu Ser Pro Gly Leu Pro Ala Met Gly Gly Pro Gly Pro Gly
    290                 295                 300

Pro Cys Glu Asp Pro Ala Gly Ala Gly Gly Ala Gly Ala Gly Gly Ser
305                 310                 315                 320

Glu Pro Leu Val Thr Val Thr Val Gln Cys Ala Phe Thr Val Ala Leu
                325                 330                 335

Arg Ala Arg Arg Gly Ala Asp Leu Ser Ser Leu Arg Ala Leu Leu Gly
            340                 345                 350

Gln Ala Leu Pro His Gln Ala Gln Leu Gly Gln Leu Ser Tyr Leu Ala
        355                 360                 365

Pro Gly Glu Asp Gly His Trp Val Pro Ile Pro Glu Glu Glu Ser Leu
    370                 375                 380

Gln Arg Ala Trp Gln Asp Ala Ala Ala Cys Pro Arg Gly Leu Gln Leu
385                 390                 395                 400

Gln Cys Arg Gly Ala Gly Gly Arg Pro Val Leu Tyr Gln Val Val Ala
                405                 410                 415

Gln His Ser Tyr Ser Ala Gln Gly Pro Glu Asp Leu Gly Phe Arg Gln
            420                 425                 430

Gly Asp Thr Val Asp Val Leu Cys Glu Glu Pro Asp Val Pro Leu Ala
        435                 440                 445

Val Asp Gln Ala Trp Leu Glu Gly His Cys Asp Gly Arg Ile Gly Ile
    450                 455                 460

Phe Pro Lys Cys Phe Val Val Pro Ala Gly Pro Arg Met Ser Gly Ala
465                 470                 475                 480

Pro Gly Arg Leu Pro Arg Ser Gln Gln Gly Asp Gln Pro
                485                 490

<210>  12

<211>  203

<212>  PRT

<213>  Homo sapiens

<220>

<221>  MISC_FEATURE

<223>  Description of sequence: amino acid sequence of D-Tat-Puma (human
       )

<220>

<221>  MISC_FEATURE

<222>  (10)..(10)

<223>  Xaa = Gly or deleted

<220>

<221>  MOD_RES

<222>  (1)..(10)

<223>  amino acids 1 to 10 are D-amino acids

<400>  12

Arg Arg Arg Gln Arg Arg Lys Lys Arg Gly Met Ala Arg Ala Arg Gln
1               5                   10                  15

Glu Gly Ser Ser Pro Glu Pro Val Glu Gly Leu Ala Arg Asp Gly Pro
            20                  25                  30

Arg Pro Phe Pro Leu Gly Arg Leu Val Pro Ser Ala Val Ser Cys Gly
        35                  40                  45

Leu Cys Glu Pro Gly Leu Ala Ala Ala Pro Ala Ala Pro Thr Leu Leu
    50                  55                  60

Pro Ala Ala Tyr Leu Cys Ala Pro Thr Ala Pro Pro Ala Val Thr Ala
65                  70                  75                  80

Ala Leu Gly Gly Ser Arg Trp Pro Gly Gly Pro Arg Ser Arg Pro Arg
                85                  90                  95

Gly Pro Arg Pro Asp Gly Pro Gln Pro Ser Leu Ser Leu Ala Glu Gln
            100                 105                 110

His Leu Glu Ser Pro Val Pro Ser Ala Pro Gly Ala Leu Ala Gly Gly
        115                 120                 125

Pro Thr Gln Ala Ala Pro Gly Val Arg Gly Glu Glu Glu Gln Trp Ala
    130                 135                 140

Arg Glu Ile Gly Ala Gln Leu Arg Arg Met Ala Asp Asp Leu Asn Ala
145                 150                 155                 160

Gln Tyr Glu Arg Arg Arg Gln Glu Glu Gln Gln Arg His Arg Pro Ser
                165                 170                 175

Pro Trp Arg Val Leu Tyr Asn Leu Ile Met Gly Leu Leu Pro Leu Pro
            180                 185                 190

Arg Gly His Arg Ala Pro Glu Met Glu Pro Asn
        195                 200

<210>  13

<211>  208

<212>  PRT

<213>  Homo sapiens


<220>

<221>  MISC_FEATURE

<223>  Description of sequence: amino acid sequence of D-Tat-Bim (human)

(transcript variant 1)

<220>
<221> MISC_FEATURE
<222> (10)..(10)
<223> Xaa = Gly or deleted

<220>
<221> MOD_RES
<222> (1)..(10)
<223> amino acids 1 to 10 are D-amino acids

<400> 13

Arg Arg Arg Gln Arg Arg Lys Lys Arg Gly Met Ala Lys Gln Pro Ser
1               5                   10                  15

Asp Val Ser Ser Glu Cys Asp Arg Glu Gly Arg Gln Leu Gln Pro Ala
            20                  25                  30

Glu Arg Pro Pro Gln Leu Arg Pro Gly Ala Pro Thr Ser Leu Gln Thr
        35                  40                  45

Glu Pro Gln Gly Asn Pro Glu Gly Asn His Gly Gly Glu Gly Asp Ser
    50                  55                  60

Cys Pro His Gly Ser Pro Gln Gly Pro Leu Ala Pro Pro Ala Ser Pro
65                  70                  75                  80

Gly Pro Phe Ala Thr Arg Ser Pro Leu Phe Ile Phe Met Arg Arg Ser
                85                  90                  95

Ser Leu Leu Ser Arg Ser Ser Ser Gly Tyr Phe Ser Phe Asp Thr Asp
            100                 105                 110

Arg Ser Pro Ala Pro Met Ser Cys Asp Lys Ser Thr Gln Thr Pro Ser
        115                 120                 125

Pro Pro Cys Gln Ala Phe Asn His Tyr Leu Ser Ala Met Ala Ser Met
    130                 135                 140

Arg Gln Ala Glu Pro Ala Asp Met Arg Pro Glu Ile Trp Ile Ala Gln

145                 150                 155                 160

Glu Leu Arg Arg Ile Gly Asp Glu Phe Asn Ala Tyr Tyr Ala Arg Arg
                165                 170                 175

Val Phe Leu Asn Asn Tyr Gln Ala Ala Glu Asp His Pro Arg Met Val
            180                 185                 190

Ile Leu Arg Leu Leu Arg Tyr Ile Val Arg Leu Val Trp Arg Met His
        195                 200                 205

<210>  14

<211>  170

<212>  PRT

<213>  Homo sapiens

<220>

<221>  MISC_FEATURE

<223>  Description of sequence: amino acid sequence of D-Tat-Bik (human)

<220>

<221>  MISC_FEATURE

<222>  (10)..(10)

<223>  Xaa = Gly or deleted

<220>

<221>  MOD_RES

<222>  (1)..(10)

<223>  amino acids 1 to 10 are D-amino acids

<400>  14
   Arg Arg Arg Gln Arg Arg Lys Lys Arg Gly Met Ser Glu Val Arg Pro
   1               5                   10                  15
   Leu Ser Arg Asp Ile Leu Met Glu Thr Leu Leu Tyr Glu Gln Leu Leu
               20                  25                  30
   Glu Pro Pro Thr Met Glu Val Leu Gly Met Thr Asp Ser Glu Glu Asp
           35                  40                  45
   Leu Asp Pro Met Glu Asp Phe Asp Ser Leu Glu Cys Met Glu Gly Ser
       50                  55                  60
   Asp Ala Leu Ala Leu Arg Leu Ala Cys Ile Gly Asp Glu Met Asp Val
   65                  70                  75                  80
   Ser Leu Arg Ala Pro Arg Leu Ala Gln Leu Ser Glu Val Ala Met His
                   85                  90                  95
   Ser Leu Gly Leu Ala Phe Ile Tyr Asp Gln Thr Glu Asp Ile Arg Asp
               100                 105                 110
   Val Leu Arg Ser Phe Met Asp Gly Phe Thr Thr Leu Lys Glu Asn Ile
           115                 120                 125
   Met Arg Phe Trp Arg Ser Pro Asn Pro Gly Ser Trp Val Ser Cys Glu
       130                 135                 140
   Gln Val Leu Leu Ala Leu Leu Leu Leu Leu Ala Leu Leu Leu Pro Leu
   145                 150                 155                 160
   Leu Ser Gly Gly Leu His Leu Leu Leu Lys
                   165                 170

## Description

This invention relates to fusion proteins comprising at least one first portion (I) comprising a trafficking sequence and at least one second portion (II) comprising a full-length or partial BH3-domain sequence of a BH3-only protein, said fusion protein comprising D-enantiomeric amino acids in retro-inverso order. Furthermore, the invention relates to pharmaceutical compositions comprising said fusion proteins as well as to the use of said fusion proteins.

The cells of multicellular organisms are members of a highly organized community. The number of cells in this community is tightly regulated, not only by controlling the rate of cell division, but also by controlling the rate of cell death. If cells are no longer needed, they commit suicide by activating an intracellular death program. This significant process is called apoptosis. Apoptosis is also known as programmed cell death and is signalled by a cascade of proteins. Changes, e.g. mutations of proteins involved, that prevent this normal programmed cell death, play an essential role in many diseases, in particular cancer. Therefore, cancerous cell growth often results from defective control of cell death. Moreover, resistance to apoptosis is a key characteristic of malignant cells enabling them to increase in number and survive.

The mitochondria-dependent pathway for apoptosis is governed by Bcl-2 family proteins. Bcl-2, Bcl-xL, Bak, and many other members of the Bcl-2 family have a hydrophobic stretch of amino-acids near their carboxyl-terminus that anchors them in the outer mitochondrial membrane. In contrast, other Bcl-2 family members such as Bid, Bim, and Bad lack these membrane-anchoring domains, but target to mitochondria in response to specific stimuli. Still others have the membrane-anchoring domain, but keep it latched against the body of the protein, until stimulated to expose it (e.g. Bax).

Bcl-2 family proteins are conserved throughout metazoan evolution, with homologs found in vertebrate and invertebrate animal species. Several types of animal viruses also harbour Bcl-2 family genes within their genomes, including herpes simplex viruses implicated in cancer, such as the Epstein-Barr Virus (EBV) and Kaposi sarcoma herpesvirus (KSHV). Both pro-apoptotic and antiapoptotic Bcl-2 family proteins have been delineated. The relative ratios of anti- and pro-apoptotic Bcl-2 family proteins dictate the ultimate sensitivity or resistance of cells to various apoptotic stimuli, including growth factor deprivation, hypoxia, radiation, anticancer drugs, oxidants, and Ca²⁺ overload.

During apoptosis Bcl-2 family proteins especially regulate (promote or inhibit) the release of proteins, such as cytochrom c, Smac/DIABLO, apoptosis inducing factor, HtrA2 and endonuclease G, from mitochondria. Certain Bcl-2 family proteins (pro-apoptotic members) are major regulators of the commitment to programmed cell death as well as executioners of death signals at the mitochondrion. In summary, the Bcl-2 family consists of pro-apoptotic members triggering mitochondrial protein release and anti-apoptotic members inhibiting mitochondrial protein release. Moreover, the family can be divided into three main subclasses [(a), (b) and (c)]. Subclasses (a) and (b) are probably similar in structure to the pore-forming domains of bacterial toxins, such as the colicins and diphtheria toxin. These helical pore-like proteins include both anti-apoptotic proteins (subclass (a)) as well as multidomain pro-apoptotic proteins (subclass (b)). The anti-apoptotic members (a), e.g., Bcl-2 and Bcl-xL, display conservation throughout all four Bcl-2 Homology (BH) domains (BH1-4, also called "multidomain anti-apoptotic proteins"). Some of the proaptotic members (b), e.g., Bax and Bak, possess sequence homology in BH1-3 domains (BH1-3, also called "multidomain pro-apoptotic proteins"). In contrast, some of the proaptotic members of subclass (c), e.g., Bid, Noxa, Puma, Bik, Bim and Bad, possess sequence homology in only one domain, namely the alpha-helical BH3 domain (called "BH3-only proteins"). The BH3 domain typically consists of an amphipathic a-helix of about 15 to 20 amino-acids length, typically 16 amino acids.

BH3-only proteins (subclass (c)) act as afferent effectors of various pro-apoptotic and antiapoptotic signals (Hunt A. et al (2001) Science 293, 1784-1785). All BH3-only proteins have the ability to bind to and to functionally antagonize anti-apoptotic Bcl-2 family proteins (Hunt A. et al., *supra*). Their BH3 domain inserts into a hydrophobic crevice on the surface of antiapoptotic proteins such as Bcl-X_{L}. They transduce multiple death signals to the mitochondrion by interacting with anti-apoptotic Bcl-2 family proteins and inducing apoptosis by a mechanism that requires the presence of at least one of the multidomain pro-apoptotic Bcl-2 family proteins Bax or Bak. Moreover, some but not all BH3-only proteins have the ability to bind to multidomain pro-apoptotic members Bax or Bak.

BH3-only protein Bid, for example, can promote the pro-apoptotic assembly and function of Bax and Bak by itself, whereas other BH3-only proteins do not function as such. By using short peptides representing the alpha-helical BH3 domain of BH3-only protein Bid, it was shown that said "Bid-peptides" are capable of inducing oligomerization of Bax and Bak to release cytochrom c. This property is attributed to BH3-only protein Bim (Korsmeyer S.J. et al (2000) Cell Death Differ 7, 1166-1173). In summary, Bid binds (multidomain) pro-apoptotic Bax and Bak as well as (multidomain) anti-apoptotic Bcl-2 and Bcl-xL and is capable to activate Bax and Bak directly.

In contrast, peptides representing BH3 domains of BH3-only proteins Bad or Bik lack the ability to directly activate Bax and Bak, but retain the ability to bind to anti-apoptotic Bcl-2 family proteins (Letai A. et al (2002) Cancer Cell 2, 183-192). This is also shown by another study using peptides derived from BH3 domain of BH3-only protein Bad which stimulate Bax activity only in the presence of Bcl-xL (Moreau P.-F. et al (2003) The Journal of Biological Chemistry, vol. 278, no. 21, 19426-19435). Thus, BH3 domains of BH3-only proteins possess distinct functions. A "Bid-like" BH3 domain "activates" pro-apoptotic Bax and Bak, whereas a "Bad-like" BH3 domain "sensitizes" pro-apoptotic Bax and Bak by occupying the pocket of anti-apoptotic Bcl-2 family members. In summary, BH3 domains of BH3-only proteins represent two classes of tool compounds that initiate cell death using genetically defined pathways (Letai A. et al. (2002) Cancer Cell 2, 183-192).

As described above, BH3-only proteins were characterized in various studies and their function and mechanism of action was analysed. The results indicate that their BH3 domains may efficiently induce apoptosis in cells expressing high levels of anti-apoptotic Bcl-2 family members by triggering the release of active multidomain pro-apoptotic proteins from said anti-apoptotic proteins.

Thus, BH3 domains of BH3-only proteins are an interesting subject and form an efficient tool in the treatment of various diseases caused by defective control of programmed cell death. However, prior art does not suggest a way to directly transport said proteins or their BH3 domains, respectively, to a desired localisation in an organism or a cell. One of the main problems is to permeabilize the inner membrane of cells. Several studies include the initial polyarginine transduction approach (Wang et al, (2000), Cancer Res. 60, 1498-1502; Holinger et al. (1999), J. Biol. Chem. 274, 13298-13304). However, various amphipathic α-helical peptides, especially cationic peptides, can be attracted to negatively charged membranes, including mitochondrial membranes, where they can non-specifically disrupt the lipid matrix and membrane barrier function (Matsuzaki (2001) Biochem. Soc. Trans. 29, 598-601 ; Ellerby et al (1999), Nat. Med. 5, 1032-1038). Another approach using the construct ANT-BH3BAD, combining antennepedia (ANT) as internalization moiety and their BH3 domain of BAD, was toxic. However, toxicity was not correlated with the Bcl-2 pathway (Vieira et al. (2002), Oncogene 21, 1963-1977; Schimmer et al. (2001), Cell Death Differ. 8, 725-733).

Therefore, it is an object of the present invention to provide a system capable of directly transporting a BH3 domain of a BH3-only protein to a desired localisation in an organism.

According to the invention a fusion protein is provided which comprises at least one first portion (I) comprising a trafficking sequence and at least one second portion (II) comprising a partial or full-length BH3-domain sequence of a BH3-only protein, said fusion protein comprising D-enantiomeric amino acids in retro-inverso order in its portion (I) and, optionally, (II).

The fusion protein of the invention comprises (at least in its portion (I)) D-enantiomeric amino acids in retro-inverso order (also called "retro-inverso" protein or "retro-inverso" fusion protein). "Retro-inverso" relates to an isomer of a linear peptide in which the direction of the regular or native L-amino acid sequence is reversed and the chirality of each amino acid residue (due to the chirality of the D-amino acids in contrast to the naturally occurring L-amino acids) is inverted. The terms "fusion protein" and "protein" according to the invention are intended to have the same meaning. Retro-inverso fusion proteins according to the invention can be constructed, e.g., by synthesizing a reverse of the amino acid sequence for the corresponding native L-amino acid sequence. In D-retro-inverso enantiomeric (fusion) proteins the positions of carbonyl and amino groups in each single amide bond are inverted, thereby ensuring that the position of the side-chain groups at each alpha carbon is preserved, if compared with regular L-amino acid peptide sequences. Retro-inverso fusion proteins of the invention possess a variety of useful properties. For example, they enter cells more efficiently and are more stable (especially *in vivo*) and show lower immunogenicity than corresponding L-amino acid fusion proteins. Since naturally-occurring proteins contain L-amino acids, almost all decomposing enzymes, like proteases or peptidases, cleave peptide bonds between adjacent L-amino acids. Consequently, proteins composed of D-enantiomeric amino acids in retro-inverso form are largely resistant to proteolytic breakdown. Evidently, the retention time in cell is increased for fusion proteins of the invention as compared to their regular non-inverted L-amino acid analogues.

Portion (I) of the inventive fusion protein serves as a carrier or trafficking sequence. A "trafficking or carrier sequence" (as a portion of the inventive fusion protein) is any sequence of amino acids that directs a fusion protein or portion (II) of the fusion protein, respectively, into the cell cytoplasm or, even further, to a specific cellular destination. The trafficking sequence can e.g. direct the fusion protein to a desired location within the cell, e.g., the nucleus, the ribosome, the endoplasmatic reticulum, a lysosome, or a peroxisome. Consequently, in a preferred embodiment the trafficking sequence of the fusion protein of the invention directs the fusion protein to a defined cellular location. Anyhow, the trafficking sequence can direct the inventive fusion protein across the plasma membrane, e.g., from the extracellular cell environment through the plasma membrane into the cytoplasma thereby enhancing the cellular uptake of the fusion protein or its drug portion (II) (cargo portion), respectively, in particular by enhancing its cell permeability or by enhancing its intracellular retention time.

In general, a functionally effective portion (I) of the inventive fusion protein which comprises said trafficking sequence is defined as an amino acid sequence comprising at least 4 (contiguous) D-amino acids. Functionally effective amino sequences as comprised by portion (I) should show strong basic properties. Preferably, said functionally effective amino acid sequences contain at least 60%, preferably at least 65%, more preferably at least 70%, even more preferably at least 75%, even more preferably at least 80%, yet more preferably at least 85%, yet more preferably at least 90%, most preferably at least 96% basic amino acids, preferably arginine and/or lysine. Functionally effective portions (I) of the inventive fusion protein preferably comprise an amino acid sequence ranging from 4 to 50 D-amino acids, more preferably from 4 to 40 D-amino acids, even more preferably from 4 to 30 D-amino acids, even more preferably from 4 to about 20 D-amino acids and most preferably from 4 to about 10 D-amino acids.

Preferably, a trafficking sequence according to the invention can be derived, e.g., from a known membrane-translocating sequence. In a particularly preferred embodiment of the invention, the trafficking sequence of the fusion protein of the invention is a D-enantiomeric amino acid sequence in retro-inverso order of a TAT protein of human immunodeficiency virus (HIV). TAT is a viral protein indispensable for the HIV infection cycle. This protein is described in, e.g., WO 94/04686, U.S. Pat. Nos. 5,804,604 and 5,674,980, each incorporated herein by reference. Portion (I) of the fusion protein of the invention is linked to a portion (II), whereby portion (I) comprises some or all of the 86 amino acids of the Tat full-length sequence, however, in its retro-inverso D-form. In particular, a functionally effective portion (I) of an inventive fusion protein that has fewer than 86 amino acids can be used, which still exhibits improved cell uptake activity and, optionally, has additional localization information for entry into the cell nucleus. Tat sequence segments responsible for mediating entry and uptake into cells can be defined using known techniques (see, e.g., Franked et al., Proc. Natl. Acad. Sci, USA 86: 7397-7401 (1989). Preferably, portion (I) of the fusion protein of the invention comprises the basic region (amino acids 48-57 or 49-57, respectively) of TAT and does not comprise TAT's cysteine-rich region (amino acids 22-36) as well as the exon 2-encoded carboxy-terminal domain (amino acids 73-86) of the naturally-occuring TAT protein. The reverse of Tat's basic region (aa 57 to 48 or 57 to 49, composed of D-amino acids) may be used as portion (I) or part of portion (I) of an inventive fusion protein. Sequences containing the reverse of Tat's basic region or consisting of the reverse are herein referred to as D-Tat sequence.

Therefore, a preferred functionally effective portion (I) of the inventive fusion protein comprises a D-TAT sequence, e.g. NH₂-RRRQRRKKRG-COOH or NH₂-RRRQRRKKR-COOH (both of which retro-inverso sequences of the above-defined naturally occurring Tat basic region), or comprises sequence variants according to generic D-TAT formula NH₂₋XXXAXXXXX-COOH, wherein "X" relates to amino acid arginine or lysine and "A" relates to any non-basic amino acid. "A" is preferably located at position 4 of the above-given general formula. However, "A" may alternatively be positioned at any other intrasequential position, i.e. at position 2, 3, 5, 6, 7, or 8 instead of position 4. Moreover, the above-given general formula may be modified introducing additional non-basic residues by substituting one to four basic residues of the general formula with non-basic residues. In accordance with the above, portion (I) sequences are exclusively composed of D-amino acids in order to exhibit the same structural side chain pattern as the corresponding non-inverted sequences composed of L-amino acids.

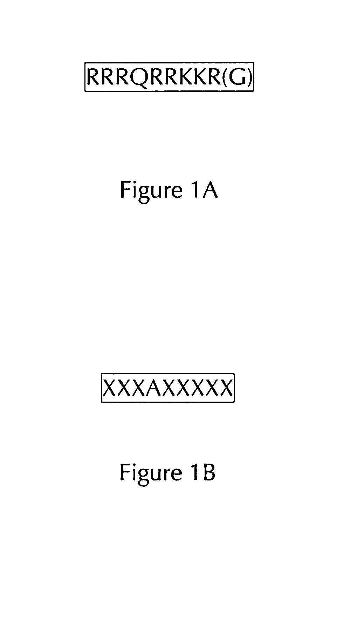

In a particularly preferred embodiment of the invention portion I comprises trafficking sequences with the amino acid of figure 1A or figure 1B.

In another embodiment functionally effective portions (I) of the inventive fusion protein comprise a generic amino acid sequence of the following general formulae: NH₂₋AmXAoXAn-COOH, NH₂-AmXXXAn-COOH, NH₂-AmXXAXAn-COOH, NH₂₋AmXAXXAn-COOH, or NH₂-XAAX-COOH, wherein "X" relates to amino acid arginine or lysine, "A" relates to any non-basic amino acid, "m" is an integer from zero to fourteen, "n" is an integer, independent from m, between zero and fourteen and "o" is an integer, independent from m and n, between zero and five. It has to be noted that the general formulae of the above trafficking sequences, according to the invention, are exclusively composed of D-enantiomeric amino acids.

Specific examples for functionally effective portions (I) of the inventive fusion protein contain or may consist of the following sequences showing strong basic properties:
NH₂-KTRR-COOH, NH₂-RLKR-COOH, NH₂-KPRR-COOH, NH₂-KRFQR-COOH, NH₂-GRIRR-COOH, NH₂-NIGRRRN-COOH, NH₂-PAGRNGR-COOH, NH₂-RPRR-COOH, NH₂-GKRRG-COOH, NH₂-KRRE-COOH, NH₂-RQKRGGS-COOH, NH₂₋RKSR-COOH, NH₂-RGSRR-COOH, NH₂-RRQK-COOH, NH₂-RARKG-COOH, NH₂₋RGRK-COOH, NH₂-RRRLS-COOH, NH₂-RPRRLSP-COOH, NH₂-RGPKY-COOH, NH₂-RPKRGMG-COOH, NH₂-GVRRR-COOH, NH₂-GYKKVGFSR-COOH, NH₂₋KFSRLSK-COOH, NH₂-RRVR-COOH, NH₂-RRSRP-COOH, NH₂-RRRM-COOH, NH₂₋KSMALTRKGGY-COOH, NH₂-RSRRG-COOH, NH₂-KMNPLPY-COOH. It has to be noted that that the above trafficking sequences, according to the invention, are exclusively composed of D-enantiomeric amino acids.

Portion (I) may comprise just one (i.e., continuous) basic cell membrane translocation sequence (D-retro-inverso form) analogous to the corresponding (naturally-occurring) translocation sequence, e.g. a D-Tat sequence as disclosed above. Alternatively, portion (I) may comprise two or more amino acid sequences in retro-inverso order which correspond to identical or different translocation sequences with strong basic properties. These retro-inverso D-form translocation motifs are synthesized on the basis of naturally-occurring protein(s) by using D-amino acids and by reverting its amino acid sequence order. In other words, an inventive fusion protein may comprise e.g. in its portion (I) a combination of two or more translocation sequences all of which are synthesized as retro-inverso D-form sequences. Typically, this combination of translocation motifs does not occur in any native protein sequence (as L-form). These two or more translocation sequences of portion (I) may be linked together with or without a linker sequence. If a linker sequence is desired, its length will preferably range from 2 to 20 amino acids.

As disclosed above, portion (I) may contain (one or more) D-enantiomeric amino acid translocation motifs (in retro-inverso order as compared to the native sequence), which are provided on the basis of its corresponding naturally-occurring L-form analog. Alternatively, portion (I) may contain D amino acid strings (in retro-inverso order) of L-form sequences, which are sequentially modified compared to the naturally occurring protein sequences (also defined as "derivatives" herein). These portion (I) derivatives (being provided in retro-inverso D-form, in other words the derivative D-form is an analog of the derivative L-form) typically possess carrier properties with uptake activity into the cell (or even, preferably, into the cell nucleus) that is substantially similar to that of the corresponding naturally-occurring protein, even though their sequence is not identical with the naturally occurring protein sequence.

In addition, sugar moieties and/or lipid moieties, e.g. cholesterol or other lipids, may be added to the inventive fusion protein, in particular to portion (I) of the inventive fusion protein or, eventually to any other portion, in order to further increase the membrane solubility of the fusion protein, e.g. to one or both termini of portion (I) to provide local lipophilicity at one or both termini. Alternatively or additionally, sugar or lipid moieties may be linked to the amino acid side chains, in particular side chains having hydroxyl and amino groups.

Portion (I) of the inventive fusion protein has to retain its cell permeability and/or its intracellular retention function. However, other functions may be added by modifications introduced into portion (I). Therefore, portion (I) can be modified, e.g., to efficiently direct the inventive fusion protein of the invention to a particular intracellular target localization. Correspondingly, portion (I) is modified such that a specific intracellular localization is awarded to portion (I) without loss of its enhanced cell permeability properties. Typically, a routing sequence for targeting the inventive fusion protein to specific cell compartments (e.g., endoplasmic reticulum, mitochondrion, gloom apparatus, lysosomal vesicles) can be introduced into portion (I). On the other hand, specific sequences which ensure cytoplasmatic localization of the inventive fusion protein may be added. E.g., portion (I) may comprise at least one further sequence which binds to one or more cytoplasmatic structure(s) in order to retain the fusion protein of the invention in the cytoplasm. Alternatively, alteration of the basic region thought to be important for nuclear localization (see, e.g., Dang and Lee (1989), J.Biol.Chem. 264:18019-18023; Hauber et al. (1989), J.Virol. 63:1181-1187; Ruben et al. (1989), J.Virol. 63:1-8) can result in a cytoplasmic location or partially cytoplasmic location of portion (I), and, therefore, of the fusion protein of the invention. Therefore, portion (I) may contain altered nuclear localization signals, which lead to cytoplasmatic localization of the inventive fusion protein.

As described above, BH3-only proteins are members of the Bcl-2 family representing regulators of apoptosis by interacting with other members of Bcl-2 family. Portion (II) of the inventive fusion protein is a amino acid sequence comprising one or more partial or full-length BH3-domain sequence(s) of a BH3-only protein (defined as a subclass of the Bcl-2 family proteins). Portion (II) may either comprise a D-retro-inverso form of the naturally occurring (full-length or partial) BH3-domain sequence, thereby ensuring that its D-amino acid side chains are positioned as in the native all-L-amino acid sequence, or, alternatively, may comprise the native all-L-amino acid sequence, both of which induce apoptosis by either interacting with at least one Bcl-2 family protein or by activating or sensitising at least one pro-apoptotic member of the Bcl-2 family.

If portion (II) of the inventive fusion protein comprises a partial BH3-domain sequence, it should have (in order to be a functionally effective) at least 4 (contiguous) D- or L-amino acids in either native or retro-inverso order (reflecting the respective fragment of at least 4 L-amino acids of a BH3-domain of a BH3-only protein). In a preferred embodiment portion (II) comprises exclusively D-amino acids. Preferred functionally effective portions (II) of the inventive fusion protein comprise a full-length or partial BH3-domain sequence composed of D-amino acids in retro-inverso order ranging from 4 to 20 D-amino acids, more preferably from 4 to 18 D-amino acids, more preferably from 4 to 15 D-amino acids, even more preferably from 4 to 13 D-amino acids, more preferably from 4 to about 10 D-amino acids, and yet more preferably from 4 to 8 D-amino acids. If partial BH3-domain sequences are used as portion (II) or part of portion (II), they have to remain functional, i.a. they are expected to retain their pro-apoptotic activity. Their functional activity can be tested by suitable assay methods, e.g. by binding assays (binding of portion II of the inventive fusion protein or of the inventive fusion protein itself to its native binding partner) or by assaying its pro-apoptotic activity by apoptosis assays.

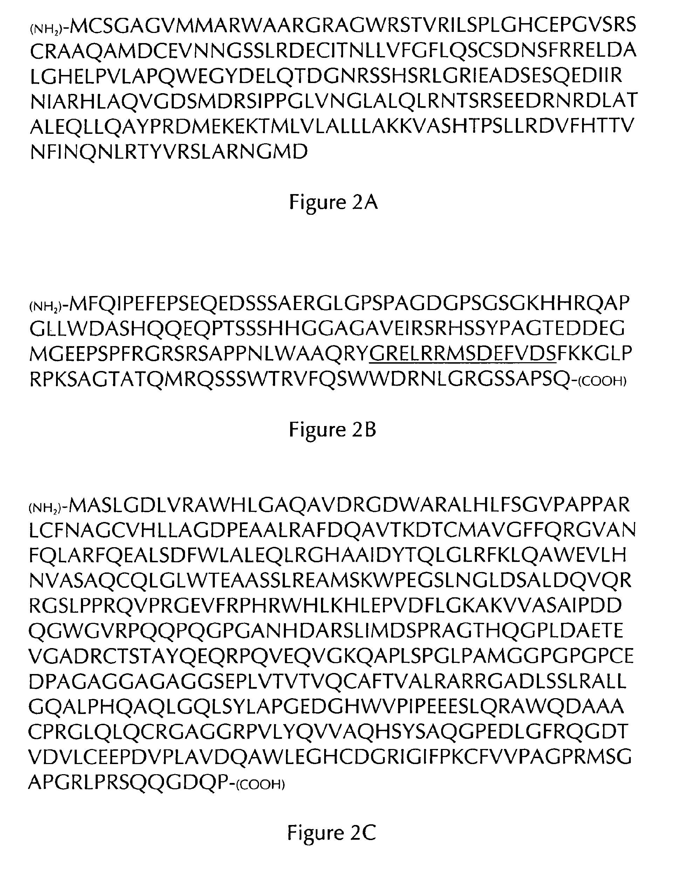

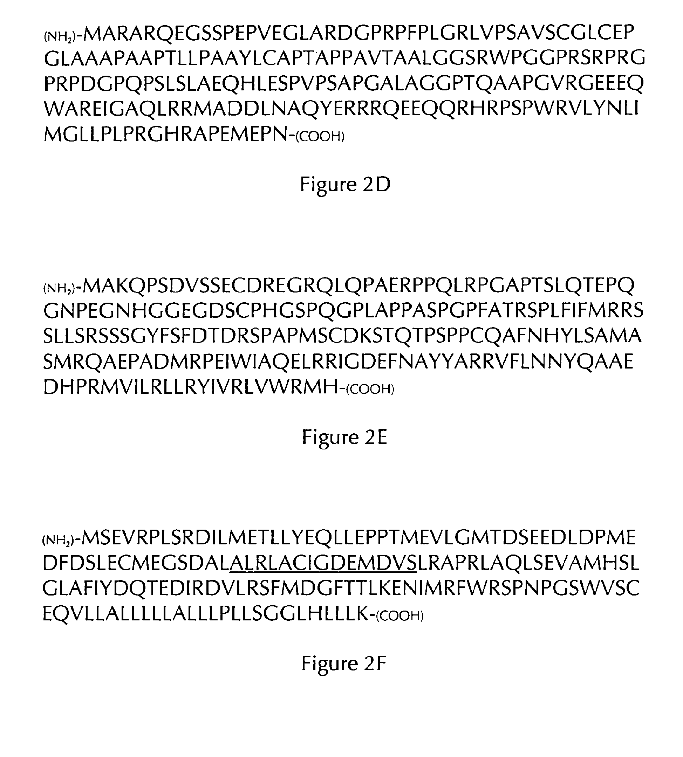

Preferably, portion (II) comprises a partial or full-length of BH3-domain sequence of a BH3-only protein selected from the group consisting of Bid, Bad, Noxa, Puma, Bim and Bik, either in its native L-form or in its retro-inverso D-form. The (full-length or partial) BH3-sequences may be selected from e.g. any mammalian BH3-only protein, in particular from the human isoforms. Accordingly, portion (II) of the inventive fusion protein comprises the amino acid sequence of figures 2A, 2B, 2C, 2D, 2E or 2F (either in its native form composed of L-amino acids as shown in figures 2A to 2F or in its retro-inverso D-form (composed of D amino acids), which means that the sequence of figure 2A to 2F have to be inverted by reverting the termini: native C-terminus is the N-terminus of the inverted form and the native N-terminus is the C-Terminus of the inverted form). Portion (II) of the inventive fusion protein may contain fragments of the native full-length BH3-only protein sequence (either in its L- or D-form), e.g. fragments of less than 50, preferably of less than 40 and even more preferably of less than 30 amino acids. These fragments of the native sequences typically comprise or at least partially (at least 7 amino acids of the BH3-domain sequence) comprise a BH3-domain sequence.

The sequence of portion (II) of the inventive fusion protein may contain one or more partial or full-length BH3-domain sequence(s) of BH3-only proteins either in its/their native L-amino acid form or in its/their inverted form being composed of D-amino acids. If portion (II) is composed of L-amino acids, it should be synthesized either by recombinant methods known to the skilled person or by peptide synthesis methods, e.g. solid phase synthesis. The L-amino acid sequence of portion (II) is afterwards coupled (by a second step) to the D-amino acid sequence of portion (I). Alternatively, chemical synthesis may allow to directly synthesize (without introducing a second coupling step) an inventive fusion protein composed of D-amino acids in its portion (I) and of L-amino acids in its portion (II). If, however, portion (II) is composed of D-amino acids as well, the fusion protein is chemically synthesized (according to the same methods as used for the synthesis of L-amino acid sequences) by using D-enantiomeric amino acids in retro-inverso order in order to invert the naturally occurring partial or full-length BH3-domain sequence and the e.g. naturally occurring trafficking sequence, e.g. the naturally occurring basic Tat-sequence.

The amino acid sequence of naturally-occurring BH3-only proteins or their fragments containing the BH3-domain and, correspondingly, their retro-inverso analogs composed of D-enantiomeric amino acids as part of portion (II) can be modified, for example, by addition, deletion and/or substitution of at least one amino acid of the naturally-occurring protein, to provide modified non-native sequences. Modifications, in particular conservative modifications, may be desired to e.g. modify the binding properties of portion (II) or modify its stability, e.g. increasing or decreasing its stability. Portion (II) with a modified primary sequence (as compared to naturally occurring BH3-domain or BH3-only protein sequences) may be produced by using the same techniques as for the synthesis of unmodified L-amino acid or D-amino acid sequences.

Fusion proteins of the invention are composed at least of portion (I) and portion (II). Moreover, the fusion protein according to the invention may comprise further portions (III), (IV), (V). These optional additional portions may award additional functions to the inventive fusion protein. The at least one further portion can be an amino acid, oligopeptide or polypeptide or an small organo-chemical compound and can be linked to the fusion protein of the invention at a suitable position, for example, the N-terminus, the C-terminus or internally coupled to amino acid side chains. Further portions (e.g, HA, HSV-Tag, His6) may render the inventive fusion protein amenable to purification and/or isolation. If desired, the fusion partner can then be removed from fusion protein of the invention (e.g., by proteolytic cleavage or other methods known in the art) at the end of the production process. Alternatively, further trafficking sequences for specific cell compartments or other functional sequences may be fused to the inventive molecule by adding an additional portion or may be incorporated into portion (I) or (II). Preferably, an additional portion (e.g. portion (III)) allows the inventive fusion protein to specifically bind to a certain cell type, e.g immune cells, hepatocytes, in particular tumor cells of organ, e.g. lymphocytes, neurons etc. Specificity is achieved by fusing ligands (e.g. ligands for extracellular portions of membrane proteins, like receptors, or antibodies directed to extracellular portions of membrane proteins, tumor cell markers), which bind to these cell markers, to the inventive fusion protein. Thereby, the inventive fusion protein may be directed specifically to target cells or tissues of an animal to be treated.

In another preferred embodiment, more than one, preferably two or three identical or non-identical, partial or full-length BH3-domain sequences may be incorporated into the inventive fusion protein either as part of portion (II) or as additional portion (e.g. (III)). In particular, partial or full-length BH3-domain sequences of different BH3-only proteins may be combined within the inventive fusion protein, e.g. Bid and Bad, Bim and Bad, Bik and Bad, Puma and Bad, Noxa and Bad, Bik and Bim, Bik and Bid, Bik and Puma, Bik and Noxa, Bid and Puma, Bid and Noxa, Bid and Bim, Bim and Noxa, Bim and Puma and Puma and Noxa. Preferably, if more an inventive fusion protein has more than one BH3-domain, the inventive fusion protein comprises a (partial or full-length) BH3-domain of a "Bid"-like and a "Bad"-like BH3-only protein. Typically, if more than one BH3-domain is present in the inventive fusion protein, both or all of them will either exhibit the retro-inverso D-form or the regular native L-form. Alternatively, an inventive fusion protein may combine D- and L-form BH3-domains.

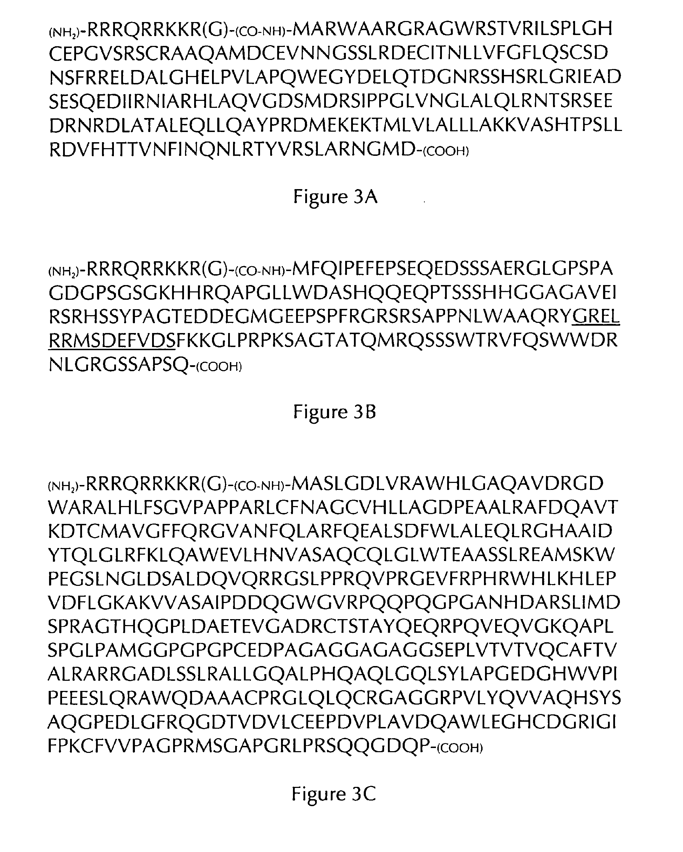

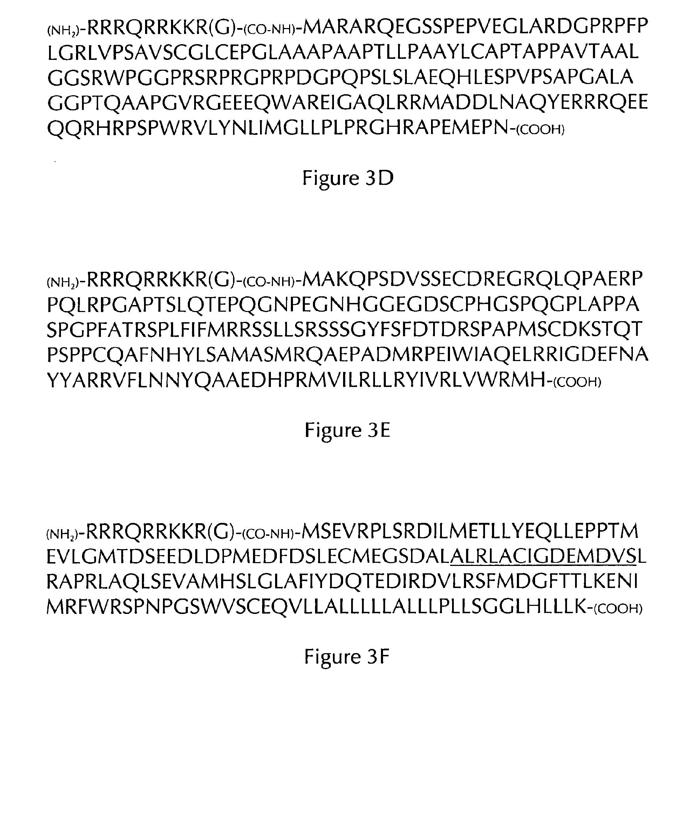

Consequently, the fusion protein according to the invention typically has a length ranging from at least 12 to about 200 amino acids or more (dependent on the additional portions and/or the length of portion (II)), preferably from 12 to about 150 amino acids, more preferably about 20 to about 100 amino acids, even more preferably about 20 to about 75 amino acids, even more preferably about 20 to about 50 amino acids. If portion (II) is composed of L-amino acids, the fusion protein according to the invention preferably has a sequence according to figures 3A, 3B, 3C, 3D, 3E or 3F. Alternatively, the fusion protein may have a trafficking sequence (being contained by portion (I)) as shown in these figures and a fragment (being contained by portion (I)) of typically less than 50 contiguous amino acids of the full-length BH3-only protein sequences shown in these figures, the fragment comprising at least partially the BH3-domain sequence of an BH3-only protein. In another embodiment of the invention, the sequences of each portion (II) shown in these figures may be composed of D-amino acids and are reversed by reversing the termini resulting in all-D-amino acid retro-inverso sequences of portion (II).

In an preferred embodiment of the invention, the at least one first portion (I) and the at least one second portion (II) of the fusion protein of the invention are linked by a covalent bond. "Covalent bond" relates to a stable chemical link between two atoms produced by sharing one or more pairs of electrons. If present, further portions (III), (TV), (V) etc., as mentioned above, can also be linked by a covalent bond either to portion (I) or to portion (II).

In general, portion (I) and portion (II) can be coupled via a linker or directly (without linker) by an amide bridge formed between N-terminal and C-terminal, respectively, amino acids of portion (I) and (II). The C-terminal amino acid of portion (I) may be linked to the N-terminal amino acid of portion (II) or the N-terminal amino acid of portion (I) may be linked to the C-terminal amino acid of portion (II). Thus, fusion proteins of the invention may have for example, a C-terminal or N-terminal trafficking sequence (portion (I)) depending upon which terminus of portion (I) is covalently linked to portion (II), whereby a linker may be added between portion (I), e.g., a sequence comprising a D-TAT sequence, and portion (II) as partial or full-length BH3-domain sequence containing portion.

If present, further portions (III), (IV), (V) etc., as mentioned above, can be coupled in an analogous manner with portion (I) or portion (II) or, optionally with each other or. Linker sequences can also be used to fuse the fusion protein of the invention with at least one other moiety/moieties as described below. Optional portions (III), (IV) etc. may be linked to the inventive fusion protein via the side chains of its D amino acids or may be attached to either terminus of the portion (I) or (II). The linkage via a side chain will preferably be based on side chain amino or hydroxyl groups, e.g. via an ester or ether linkage. It has to be noted that, according to the invention, all amino acids (of any of portions (I), (II), (III), (IV), (V) etc.,) are of D-enantiomeric amino acids, which mimic its eventually naturally occurring analogue by being linked in retro-inverso order.

If linker sequences are used to fuse portion (I) and (II) or to fuse another portion, e.g. (III) to portion (I) and/or (II), the linker sequences preferably form a flexible sequence of 5 to 50 residues, more preferably 5 to 15 residues. In a preferred embodiment the linker sequence (either an all-D- or all-L-amino acid sequence) contains at least 20%, more preferably at least 40% and even more preferably at least 50% Gly residues. Appropriate linker sequences can be easily selected and prepared by a person skilled in the art.

Portion (I) and portion (II) can also be linked by chemical coupling in any suitable manner known in the art. However, attention is drawn to the fact that many known chemical cross-linking methods are non-specific, i.e., they do not direct the point of coupling to any particular site on the transport protein (or polypeptide) or cargo moiety. Thus, the use of non-specific cross-linking agents may attack functional sites or sterically block active sites, rendering the fused proteins biologically inactive. If present, further portions (III), (TV), (V) etc., as mentioned above, can be coupled in an analogous manner to one another or to portion (I) and/or (II).

Coupling specificity can be increased by directly chemical coupling to a functional group found only once or a few times in one or both of the proteins/peptides/polypeptides to be cross-linked. An example is cystein which is the only amino acid containing a thiol group and which occurs only a few times in many proteins. Also, for example, if a protein/peptide/polypeptide contains no lysine residues, a cross-linking reagent specific for primary amines will be selective for the amino terminus of that protein/peptide/polypeptide. However, successful utilization of this approach to increase coupling specificity requires that the protein/peptide/polypeptide have the suitably rare and reactive residues in areas of the molecule that may be altered without loss of the molecule's biological activity.

Cysteine residues may be replaced when they occur in parts of a protein/peptide/polypeptide sequence where their participation in a cross-linking reaction would otherwise likely interfere with biological activity. When a cysteine residue is replaced, it is typically desirable to minimize resulting changes in protein (or polypeptide) folding. Changes in protein/peptide/polypeptide folding are minimized when the replacement is chemically and sterically similar to cysteine. Therefore, serine is preferred as a replacement for cysteine. However, a cysteine residue is introduced, introduction at or near the N- or C-terminus is preferred. Conventional methods are available for such amino acid sequence modifications, whether the protein/peptide/polypeptide of interest is produced by chemical synthesis or expression of recombinant DNA.

Coupling of the two constituents, e.g. for coupling the D-form of portion (I) to an portion (II) L-form, can be accomplished via a coupling or conjugating agent. There are several intermolecular cross-linking reagents which can be utilized, see for example, Means and Feeney, Chemical Modification of Proteins, Holden-Day, 1974, pp. 39-43. Among these reagents are, for example, N-succinimidyl 3-(2-pyridyldithio)propionate (SPDP) or N,N'-(1,3-phenylene)bismaleimide; N,N'-ethylene-bis-(iodoacetamide) or other such reagent having 6 to 11 carbon methylene bridges; and 1,5-difluoro-2,4-dinitrobenzene. Other cross-linking reagents useful for this purpose include: p,p'-difluoro-m,m'-dinitrodiphenylsulfone; dimethyl adipimidate; phenol-1,4-disulfonylchloride; hexamethylenediisocyanate or diisothiocyanate, or azophenyl-p-diisocyanate; glutaraldehyde and disdiazobenzidine. Cross-linking reagents may be homobifunctional, i.e., having two functional groups that undergo the same reaction. A preferred homobifunctional cross-linking reagent is bismaleimidohexane (BMH). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of proteins (or polypeptides) that contain cysteine residues. Cross-linking reagents may also be heterobifunctional. Heterobifunctional cross-linking agents have two different functional groups, for example an amine-reactive group and a thiol-reactive group, that will cross-link two proteins having free amines and thiols, respectively. Examples of heterobifunctional cross-linking agents are succinimidyl 4-(N-maleimidomethyl) cyclohexane-1-carboxylate (SMCC), m-maleimidobenzoyl-N-hydroxysuccinimide ester (MBS), and succinimide 4-(p-maleimidophenyl)butyrate (SMPB), an extended chain analog of MBS. The succinimidyl group of these cross-linkers reacts with a primary amine, and the thiol-reactive maleimide forms a covalent bond with the thiol of a cysteine residue. Because cross-linking reagents often have low solubility in water, a hydrophilic moiety, such as a sulfonate group, may be added to the cross-linking reagent to improve its water solubility. Sulfo-MBS and sulfo-SMCC are examples of cross-linking reagents modified for water solubility. Many cross-linking reagents yield a conjugate that is essentially non-cleavable under cellular conditions. Therefore, some cross-linking reagents contain a covalent bond, such as a disulfide, that is cleavable under cellular conditions. For example, Traut's reagent, dithiobis (succinimidylpropionate) (DSP), and N-succinimidyl 3-(2-pyridyldithio)propionate (SPDP) are well-known cleavable cross-linkers. The use of a cleavable cross-linking reagent permits the cargo moiety to separate from the transport polypeptide after delivery into the target cell. For this purpose, direct disulfide linkage may also be useful. Chemical cross-linking may also include the use of spacer arms. Spacer arms provide intramolecular flexibility or adjust intramolecular distances between conjugated moieties and thereby may help preserve biological activity. A spacer arm may be in the form of a protein (or polypeptide) moiety that includes spacer amino acids, e.g. proline. Alternatively, a spacer arm may be part of the cross-linking reagent, such as in "long-chain SPDP" (Pierce Chem. Co., Rockford, Ill., cat. No. 21651 H). Numerous cross-linking reagents, including the ones discussed above, are commercially available. Detailed instructions for their use are readily available from the commercial suppliers. A general reference on protein cross-linking and conjugate preparation is: Wong, Chemistry of Protein Conjugation and Cross-Linking, CRC Press (1991).

The present invention also encompasses fusion proteins, which mimic native functional sequences or its D-form analog, but are sequentially modified. These modified variants are defined as derivatives and are also fusion proteins of the invention. A "derivative" may comprise a derivative of portion (I) and/or portion (II) as well as portion (III), portion (TV), portion (V) etc. (if present) of the naturally occurring L-sequence or its corresponding retro-inverso D-form. It is intended to indicate a fusion protein which is derived from the native L-sequence (or its D-form analog) by substitution of one or more L- or D-amino acids at one, two or more of sites of the amino acid sequence, deletion of one or more amino acids at either or both ends of the inventive amino acid sequence or at one or more sites of the amino acid sequence, or insertion of one or more amino acids at one or more sites of the inventive amino acid sequence retaining its characteristic activity. Preferably, substitution of amino acid(s) relates to conservative substitution. Conservative substitutions typically include substitutions within the following classes: glycine and alanine; valine, isoleucine and leucine; aspartic acid and glutamic acid; asparagine and glutamine; serine and threonine; lysine and arginine; and phenylalanine and tyrosine. Thus, preferred conservative substitution groups are aspartate-glutamate; asparagine-glutamine; valine-leucine-isoleucine; alanine-valine; phenylalanine-tyrosine and lysine-arginine. By these sequence alterations of portion (I) and/or (II) (as well as portion (III), (IV), (V) etc., if present), e.g. the stability and/or effectiveness of the inventive fusion proteins can be modified, e.g. enhanced. Fusion proteins of the invention being modified as compared to their native L-form (or to its D-form analog; both L- and D-form reflecting the native sequence are also termed "parent sequences") have to remain homologous, e.g. in sequence, in function, and in antigenic character or other characteristics, with a protein having the corresponding parent sequence. It is particularly preferred that the derivatives of the native trafficking sequence being comprised in portion (I) remain functional (maintain its character as cell permeable moiety), whereas the derivatives of the native (partial or full-length) BH3-domain sequence being comprised in portion (II) maintain their pro-apoptotic property. These inventive fusion proteins derived from a native parent sequence possess altered properties being advantageous over the properties of the parent sequence (e.g. increased pH optimum, increased temperature stability etc.). It is understood that e.g. portion (I) of the inventive fusion protein may either reflect the retro-inverso D-form of the native L-form analog or may reflect a derivative of the retro-inverso D-form (an analog of the native L-form) by introduction of sequence modification(s).

Derivatives of native D- or L-form sequences (forming inventive fusion proteins) typically have substantial identity with the native amino acid sequences, e.g. as disclosed herein by figures 2A to 2F for the portion (II). Particularly preferred are amino acid sequences which have at least 60% sequence identity, preferably at least 75% sequence identity, more preferably at least 80%, still more preferably 90% sequence identity and most preferably at least 95% sequence identity thereto. Sequence identity can be measured, e.g. by using sequence analysis software (Sequence Analysis Software Package of the Genetics Computer Group, University of Wisconsin Biotechnology Center, 1710 University Avenue, Madison, Wis. 53705) with appropriate default parameters therein.

The production of native protein sequences composed of L-amino acids by recombinant techniques or by in vitro translation is well known and can be carried out following standard methods which are well known by a person skilled in the art (see e.g., Sambrook J, Maniatis T (1989) *supra*). Recombinant techniques may be used to prepare certain portions of the inventive fusion protein, e.g. portion (II), if it comprises native or derivative L-form sequences according to one embodiment of the present invention. These L-form portion (II) is then linked to the D-form portion (I) by a separate step as disclosed above. In general, the recombinant preparation of a portion of a fusion protein of the invention is carried out by either selecting the desired native DNA sequence or by modifying a native DNA sequence, by transformation of that DNA sequence into a suitable host and expression of the (modified) DNA sequence to form the desired protein sequence. The isolation of desired protein sequence can be carried out using standard methods including separating the (host) cells from the medium by centrifugation or filtration, if necessary after disruption of the cells, precipitating the proteineous components of the supernatant or filtrate by means of a salt, e. g., ammonium sulfate, followed by purification by using a variety of chromatographic procedures, e. g., ion exchange chromatography, affinity chromatography or similar art recognized procedures (see, e.g., Sambrook J, Maniatis T (1989) *supra*).

In more detail, the recombinant methods include transforming a suitable host cell with a nucleic acid or a vector provided herein which encodes the L-form of e.g. portion (II) of the fusion protein and cultivating the resultant recombinant microorganism, preferably *E.coli,* under conditions suitable for host cell growth and nucleic acid expression, e.g., in the presence of inducer, suitable media supplemented with appropriate salts, growth factors, antibiotic, nutritional supplements, etc.), whereby the nucleic acid is expressed and the encoded fusion protein is produced.

A vector comprising the nucleic acid of the native L-form of portion (II) of the fusion protein of the invention defines a nucleic acid sequence which comprises (apart from other sequences) one or more nucleic acid sequences of e.g. the L-form sequence of portion (II). A vector can be used, upon transformation into an appropriate host cell, to allow expression of said nucleic acid. The vector may be a plasmid, a phage particle or simply a potential genomic insert. Once transformed into a suitable host, the vector may replicate and function independently of the host genome, or may, under suitable conditions, integrate into the genome itself. Preferred vectors according to the invention are *E.coli* XL-Blue MRF' and pBK-CMV plasmid.

The aforementioned term "other sequences" of a vector relate to the following: In general, a suitable vector includes an origin of replication, for example, Ori p, colEl Ori, sequences which allow the inserted nucleic acid to be expressed (transcribed and/or translated) and/or a selectable genetic marker including, e.g., a gene coding for a fluorescence protein, like GFP, genes which confer resistance to antibiotics such as the p-lactamase gene from Tn3, the kanamycin-resistance gene from Tn903 or the chloramphenicol-resistance gene from Tn9.

The term "plasmid" means an extrachromosomal usually self-replicating genetic element. Plasmids are generally designated by a lower "p" preceded and/or followed by letters and numbers. The starting plasmids herein are either commercially available, publicly available on an unrestricted basis or can be constructed from available plasmids in accordance with the published procedures. In addition, equivalent plasmids to those described are known to a person skilled in the art. The starting plasmid employed to prepare a vector of the present invention may be isolated, for example, from the appropriate *E. coli* containing these plasmids using standard procedures such as cesium chloride DNA isolation.

A suitable vector also relates to a (recombinant) DNA cloning vector as well as to a (recombinant) expression vector. A DNA cloning vector refers to an autonomously replicating agent, including, but not limited to, plasmids and phages, comprising a DNA molecule to which one or more additional nucleic acids of e.g portion (II) of the fusion protein in its native L-form have been added. An expression vector relates to any DNA cloning vector recombinant construct comprising a nucleic acid sequence to be expressed operably linked to a suitable control sequence capable of effecting the expression and to control the transciption of the inserted nucleic acid in a suitable host. Such plasmids may also be readily modified to construct expression vectors that produce the desired sequence in a variety of organisms, including, for example, *E. coli,* Sf9 (as host for baculovirus), *Spodoptera* and *Saccharomyces*. The literature contains techniques for constructing AV12 expression vectors and for transforming AV12 host cells. U.S. Pat. No. 4,992,373, herein incorporated by reference, is one of many references describing these techniques.

"Operably linked" means that the nucleic acid sequence is linked to a control sequence in a manner which allows expression (e. g., transcription and/or translation) of the nucleic acid sequence. "Transcription" means the process whereby information contained in a nucleic acid sequence of DNA is transferred to complementary RNA sequence

"Control sequences" are well known in the art and are selected to express the nucleic acid of the L-form of the fusion protein of the invention and to control the transcription. Such control sequences include, but are not limited to a polyadenylation signal, a promoter (e.g., natural or synthetic promotor) or an enhancer to effect transcription, an optional operator sequence to control transcription, a locus control region or a silencer to allow a tissue-specific transcription, a sequence encoding suitable ribosome-binding sites on the mRNA, a sequence capable to stabilize the mRNA and sequences that control termination of transcription and translation. These control sequences can be modified, e.g., by deletion, addition, insertion or substitution of one or more nucleic acids, whereas saving their control function. Other suitable control sequences are well known in the art and are described, for example, in Goeddel (1990), Gene Expression Technology:Methods in Enzymology 185, Academic Press, San Diego, CA.

Especially a high number of different promoters for different organism is known. For example, a preferred promoter for vectors used in *Bacillus subtilis* is the AprE promoter; a preferred promoter used in *E. coli* is the T7/Lac promoter, a preferred promoter used in *Saccharomyces cerevisiae* is PGK1, a preferred promoter used in *Aspergillus niger* is glaA, and a preferred promoter used in *Trichoderma reesei (reesei)* is cbhI. Promoters suitable for use with prokaryotic hosts also include the beta-lactamase (vector pGX2907 (ATCC 39344) containing the replicon and beta-lactamase gene) and lactose promoter systems (Chang et al. (1978), Nature (London), 275:615; Goeddel et al. (1979), Nature (London), 281:544), alkaline phosphatase, the tryptophan (trp) promoter system (vector pATH1 (ATCC 37695) designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable a person skilled in the art to ligate them to DNA encoding the desired protein sequence, e.g. portion (II) of the inventive fusion protein in its L-forms, using linkers or adapters to supply any required restriction sites. Promoters for use in bacterial systems also will contain a Shine-Dalgarno sequence operably linked to the DNA encoding the desired protein sequence to be used as (part of) the inventive fusion protein.

Useful expression vectors, for example, may consist of segments of chromosomal, non-chromosomal and synthetic DNA sequences such as various known derivatives or fragments of SV40 and known bacterial plasmids, e.g., plasmids from *E. coli* including col E1, pBK, pCR1, pBR322, pMb9, pUC 19 and their derivatives, wider host range plasmids, e.g., RP4, phage DNAs e.g., the numerous derivatives of phage lambda, e.g., NM989, and other DNA phages, e.g., M13 and filamentous single stranded DNA phages, yeast plasmids, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors described above are known in the art and are described generally in, for example, Sambrook J, Maniatis T (1989) *supra*.

Suitable "cells" or "host cells" comprising an aforementioned vector or a nucleic acid of the e.g. portion (II) L-form of the fusion protein of the invention may serve as a host and expression vehicle for the sequence to be expressed. The host cell can be e.g., a prokaryotic, an eukaryotic or an archaeon cell. Host cells comprise (for example, as a result of transformation, transfection or tranduction) a vector or nucleic acid as described herein include, but are not limited to, bacterial cells (e.g., R. *marinus, E. coli, Streptomyces, Pseudomonas, Bacillus, Serratia marcescens, Salmonella typhimurium*), fungi including yeasts (e. g., *Saccharomycies cerevisie, Pichia pastoris)* and molds (e.g., *Aspergillus sp*.), insect cells (e.g., Sf9) or mammalian cells (e.g., COS, CHO). Preferably, host cells means the cells of *E. coli.* In general, a host cell may be selected modulating the expression of inserted sequences of interest or modifying or processing expressed proteins encoded by the sequences in the specific manner desired. Appropriate cells or cell lines or host systems may thus be chosen to ensure the desired modification and processing of the foreign protein is achieved. For example, protein expression within a bacterial system can be used to produce an unglycosylated core protein, whereas expression within mammalian cells ensures "native" glycosylation of a heterologous protein.

Eukaryotic host cells are not limited to use in a particular eukaryotic host cell. A variety of eukaryotic host cells are available, e.g., from depositories such as the American Type Culture Collection (ATCC) and are suitable for use with vectors as described above. The choice of a particular host cell depends on the character of the expression vector used. Eukaryotic host cells include mammalian cells as well as yeast cells. The imperfect fungus *Saccharomyces cerevisiae* is the most commonly used eukaryotic microorganism, although a number of other strains are commonly available. For expression in *Saccharomyces sp*., the plasmid YRp7 (ATCC-40053), for example, is commonly used (see. e,g., Stinchcomb L. et al. (1979) Nature, 282:39; Kingsman J. al. (1979), Gene, 7:141; S. Tschemper et al. (1980), Gene, 10:157). This plasmid already contains the trp gene which provides a selectable marker for a mutant strain of yeast lacking the ability to grow in tryptophan.

Suitable promoting sequences for use with yeast hosts include the promoters for 3-phosphoglycerate kinase (found on plasmid pAP12BD (ATCC 53231) and described in U.S. Pat. No. 4,935,350, issued Jun. 19, 1990, herein incorporated by reference) or other glycolytic enzymes such as enolase (found on plasmid pAC1 (ATCC 39532)), glyceraldehyde-3-phosphate dehydrogenase (derived from plasmid pHcGAPC1 (ATCC 57090, 57091)), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase, as well as the alcohol dehydrogenase and pyruvate decarboxylase genes of Zymomonas mobilis (U.S. Pat. No. 5,000,000 issued Mar. 19, 1991, herein incorporated by reference). Other yeast promoters, which are inducible promoters, having the additional advantage of their transcription being controllable by varying growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV (ATCC 39475) and described in U.S. Pat. No. 4,840,896, herein incorporated by reference), glyceraldehyde 3-phosphate dehydrogenase, and enzymes responsible for maltose and galactose (e.g. GAL1 found on plasmid pRY121 (ATCC 37658)) utilization. Yeast enhancers such as the UAS Ga1 from Saccharomyces cerevisiae (found in conjuction with the CYC1 promoter on plasmid YEpsec--hI1beta ATCC 67024), also are advantageously used with yeast promoters.

An aforementioned vector can be introduced into a host cell using any suitable method (e.g., transformation, electroporation, transfection using calcium chloride, rubidium chloride, calcium phosphate, DEAEdextran or other substances, microprojectile bombardment, lipofection, infection or transduction). Transformation relates to the introduction of DNA into an organism so that the DNA is replicable, either as an extrachromosomal element or by chromosomal integration. Methods of transforming bacterial and eukaryotic hosts are well known in the art. Numerous methods, such as nuclear injection, protoplast fusion or by calcium treatment are summerized in Sambrook J, Maniatis T (1989) *supra.* Transfection refers to the taking up of a vector by a host cell whether or not any coding sequences are in fact expressed. Successful transfection is generally recognized when any indication or the operation or this vector occurs within the host cell.

In another embodiment of the present invention, portion (II) of the inventive fusion protein does not correspond to the native L-form or a derivative thereof, but to a retro-inverso D-form sequence composed of D-amino acids and reversed in its order. These D-form sequences of portion (II) (as with the D-form sequence of portion (I)) are typically not amenable by recombinant techniques and are preferably synthesized by peptide synthesis technology, e.g. solid phase synthesis, using D-amino acids instead of L-amino acids to obtain a D-amino acid sequence. However, it has to be noted that the amino acid order has to be reversed for he synthesis (as compared to the L-form analog).

Correspondingly, all amino acid sequences of the invention (either D- or L-form sequences) can be constructed by chemical methods well known in the art, including solid phase protein synthesis, or recombinant methods. Both methods are described in U.S. Pat. No. 4,617,149, the entirety of which is herein incorporated by reference. The principles of solid phase chemical synthesis of fusion proteins are well known in the art and are described by, e.g., Dugas H. and Penney C. (1981), Bioorganic Chemistry, pages 54-92. For examples, proteins and peptides may be synthesized by solid-phase methodology utilizing an Applied Biosystems 430A peptide synthesizer (commercially available from Applied Biosystems, Foster City, Calif.) and synthesis cycles supplied by Applied Biosystems. Protected amino acids, such as t-butoxycarbonyl-protected amino acids, and other reagents are commercially available from many chemical supply houses. Sequential t-butoxycarbonyl chemistry using double couple protocols are applied to the starting p-methyl benzhydryl amine resins for the production of C-terminal carboxamides. For the production of C-terminal acids, the corresponding pyridine-2-aldoxime methiodide resin is used. Asparagine, glutamine, and arginine are coupled using preformed hydroxy benzotriazole esters. The following side chain protection may be used:
Arg, Tosyl; Asp, cyclohexyl; Glu, cyclohexyl; Ser, Benzyl; Thr, Benzyl; Tyr, 4-bromo carbobenzoxy.
Removal of the t-butoxycarbonyl moiety (deprotection) may be accomplished with trifluoroacetic acid (TFA) in methylene chloride. Following completion of the synthesis the proteins or peptides may be deprotected and cleaved from the resin with anhydrous hydrogen fluoride containing 10% meta-cresol. Cleavage of the side chain protecting group(s) and of the (fusion) protein from the resin is carried out at zero degrees centigrade or below, preferably -20°C. for thirty minutes followed by thirty minutes at 0 °C. After removal of the hydrogen fluoride, the (fusion) protein/resin is washed with ether, and the (fusion) protein extracted with glacial acetic acid and then lyophilized. Purification is accomplished by size-exclusion chromatography on a Sephadex G-10 (Pharmacia) column in 10% acetic acid. As explained above peptides composed of L-amino acids or D-amino acids are synthesized according to the same general technology.

Another embodiment of the present invention provides a pharmaceutical composition which comprises a fusion protein of the invention and optionally a pharmaceutically acceptable carrier, adjuvant and/or vehicle. This pharmaceutical composition is intended for the treatment of diseases which are at least in part caused by changes (e.g. mutations) in normal programmed cell death (apoptosis). In particular, such changes prevent (all or in part) normal programmed cell death (apoptosis). Preferably, these diseases include cancer, e.g., Hodgkin lymphoma, non-Hodgkin lymphoma, histocytic lymphoma, cancers of the brain (glioblastomas), ovarian, genitourinary tract, colon, liver, colorectal tract, pancreas, breast, prostate, lymphatic system, stomach, larynx and lung, including lung adenocarcinoma and small cell lung cancer and/or skin, e.g. melanoma or non-melanoma skin cancer, including basal cell and squamous cell carcinomas as well as psoriasis, Behcet's syndrome, pemphigus vulgaris.

A "pharmaceutically acceptable carrier, adjuvant, or vehicle" according to the invention refers to a non-toxic carrier, adjuvant or vehicle that does not destroy the pharmacological activity of the fusion protein with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, polyethylene glycol and wool fat.

The pharmaceutical composition of the present invention may be administered parenterally or non-parenterally (e.g. orally).

The term "parenteral" as used herein includes subcutaneous, intravenous, intramuscular, intra-articular, intra-synovial, intrasternal, intrathecal, intrahepatic, intralesional and intracranial injection or infusion techniques. Preferably, the pharmaceutical compositions are administered orally, intraperitoneally or intravenously. Sterile injectable forms of the pharmaceutical compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

If administered orally, the pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavouring or colouring agents may also be added. Additionally, standard pharmaceutical methods can be employed to control the duration of action. These are well known in the art and include control release preparations and can include appropriate macromolecules, for example polymers, polyesters, polyaminoacids, polyvinylpyrrolidone, ethylenevinylacetate, methyl cellulose, caraboxymethyl cellulose or protamine sulfate. The concentration of macromolecules as well as a the methods of incorporation can be adjusted in order to control release. Additionally, the agent can be incorporated into particles of polymeric materials such as polyesters, polyaminoacids, hydrogels, poly (lactic acid) or ethylenevinylacetate copolymers. In addition to being incorporated, these agents can also be used to trap the compound in microcapsules.

Further administration forms are e.g. by inhalation spray, topically, rectally, nasally, bucally, vaginally, or via an implanted reservoir, some of which are described in the following in more detail.

Accordingly, the pharmaceutical compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The pharmaceutical compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. Topical application for the lower intestinal tract can be effected in a rectal suppository formulation (see above) or in a suitable enema formulation. Topically-transdermal patches may also be used. For topical applications, the pharmaceutical compositions may be formulated in a suitable ointment containing the active component suspended or dissolved in one or more carriers. Carriers for topical administration of the fusion proteins of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active fusion proteins suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutical compositions may be formulated as micronized suspensions in isotonic, pH adjusted sterile saline, or, preferably, as solutions in isotonic, pH adjusted sterile saline, either with or without a preservative such as benzylalkonium chloride.

Alternatively, for ophthalmic uses, the pharmaceutical compositions may be formulated in an ointment such as petrolatum.

The pharmaceutical compositions of this invention may also be administered by nasal aerosol or inhalation. Such pharmaceutical compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutical compositions of this invention are formulated for oral administration.

The amount of the fusion protein(s) of the present invention that may be combined with carriers, adjuvants and vehicles to produce a pharmaceutical composition in a single dosage form will vary depending upon the host treated, the particular mode of administration. Preferably, the pharmaceutical compositions should be formulated so that a dosage of between 0.01-100 mg/kg body weight/day of the inhibitor can be administered to a patient receiving these compositions. Preferred dosages range from 0.1 - 5 mg/kg body weight/day, even further preferred dosages from 1 - 5 mg/kg body weight/day.

Capsules: Capsules are prepared by filling standard two-piece hard gelatin capsulates each with 100 milligram of powdered active ingredient, 175 milligrams of lactose, 24 milligrams of talc and 6 milligrams magnesium stearate. Soft Gelatin Capsules: A mixture of active ingredient in soybean oil is prepared and injected by means of a positive displacement pump into gelatin to form soft gelatin capsules containing 100 milligrams of the active ingredient. The capsules are then washed and dried. Tablets: Tablets are prepared by conventional procedures so that the dosage unit is 100 milligrams of active ingredient. 0.2 milligrams of colloidal silicon dioxide, 5 milligrams of magnesium stearate, 275 milligrams of microcrystalline cellulose, 11 milligrams of cornstarch and 98.8 milligrams of lactose. Appropriate coatings may be applied to increase palatability or to delay absorption. Injectable: A parenteral composition suitable for administration by injection is prepared by stirring 1.5% by weight of active ingredients in 10% by volume propylene glycol and water. The solution is made isotonic with sodium chloride and sterilized. Suspension: An aqueous suspension is prepared for oral administration so that each 5 millimeters contain 100 milligrams of finely divided active ingredient, 200 milligrams of sodium carboxymethyl cellulose, 5 milligrams of sodium benzoate, 1.0 grams of sorbitol solution U.S.P. and 0.025 millimeters of vanillin.

It has to be noted that a specific dosage and treatment regimen for any particular patient will depend upon a variety of factors, including the activity of the specific fusion protein employed, the age, body weight, general health, sex, diet, time of administration, rate of excretion, drug combination, and the judgment of the treating physician and the severity of the particular disease being treated. The amount of a fusion protein of the present invention in the pharmaceutical composition will also depend upon the particular fusion protein in the composition.

A further embodiment of the invention relates to the use of a fusion protein of the invention for the treatment or for the preparation of a pharmaceutical composition for the treatment and/or prophylaxis of diseases or to therapeutic methods for the treatment of conditions caused by defective apoptosis, cancer, e.g., Hodgkin lymphoma, non-Hodgkin lymphoma, histocytic lymphoma, cancers of the brain (glioblastomas), ovarian, genitourinary tract, colon, liver, colorectal tract, pancreas, breast, prostate, lymphatic system, stomach, larynx and lung, including lung adenocarcinoma and small cell lung cancer and/or skin, e.g. melanoma or non-melanoma skin cancer, including basal cell and squamous cell carcinomas as well as psoriasis, Behcet's syndrome, pemphigus vulgaris.

The following figures and examples are thought to illustrate the invention and should not be constructed to limit the scope of the invention thereon. All references cited by the disclosure of the present application are hereby incorporated in their entirety by reference.

### Figures

- **Figure 1A**: shows the amino acid sequence of the basic region of the Tat protein in its retro-inverso D-form. Therefore, all amino acids shown are D-enantiomeric amino acids. The sequence of figure 1A is an examples for a D-TAT (SEQ ID NO:1).
- **Figure 1B**: shows the amino acid sequence of generic D-TAT (SEQ ID NO:2). It is referred to the description of figure 1A.
- **Figure 2A**: shows the native L-amino acid sequence of Bid (transcript variant 1, SEQ ID NO:3), human isoform.
- **Figure 2B**: shows the native L-amino acid sequence of Bad (SEQ ID NO:4); human isoform; the BH3 domain is underlined.
- **Figure 2C**: shows the native L-amino acid sequence of Noxa (SEQ ID NO:5), human isoform.
- **Figure 2D**: shows the native L-amino acid sequence of Puma (SEQ ID NO:6), human isoform.
- **Figure 2E**: shows the native L-amino acid sequence of Bim (transcript variant 1, SEQ ID NO:7), human isoform.
- **Figure 2F**: shows the native L-amino acid sequence of Bik (SEQ ID NO:8); human isoform; the BH3 domain is underlined.
- **Figure 3A**: shows the amino acid sequence of D-TAT-Bid (transcript variant 1, SEQ ID NO:9) - an example of an inventive fusion protein, whereby the Bid fulllength sequence (according to the invention, portion (II) of the inventive fusion protein) is depicted in its native L-form (as with the BH3-only protein sequences of the following figure 3B to 3F) and portion (I) in its inverted form composed of D-amino acids (D-Tat). In order to depict the inventive fusion protein as retro-inverso all-D-amino acid sequence, portion (II) has to be inverted.
- **Figure 3B**: shows the amino acid sequence of D-TAT-Bad (SEQ ID NO:10); the BH3 domain is underlined.
- **Figure 3C**: shows the amino acid sequence of D-TAT-Noxa (SEQ ID NO:11).
- **Figure 3D**: shows the amino acid sequence of D-TAT-Puma (SEQ ID NO:12).
- **Figure 3E**: shows the amino acid sequence of D-TAT-Bim (transcript variant 1, (SEQ ID NO:13).
- **Figure 3F**: shows the amino acid sequence of D-TAT-Bik (SEQ ID NO:14); the BH3 domain is underlined.

## Claims

1. A fusion protein comprising at least one first portion (I) comprising a trafficking sequence and at least one second portion (II) comprising a partial or full-length BH3-domain sequence of a BH3-only protein, said fusion protein comprising D-enantiomeric amino acids in its portion (I) in retro-inverso order.

2. The fusion protein of claim 1, wherein the at least one first portion (I) and the at least one second portion (II) are linked by a covalent bond.

3. The fusion protein of any of claims 1 or 2, wherein portion (I) comprises a trafficking sequence which directs the fusion protein to a defined cellular location.

4. The fusion protein of any of claims 1 to 3, wherein portion (I) comprises a trafficking sequence which enhances cellular uptake of the fusion protein, in particular by enhancing cell permeability.

5. The fusion protein of claim 1 to 4, wherein portion (I) comprises a trafficking sequence which is a D-TAT sequence of a TAT protein of a human immunodeficiency virus.

6. The fusion protein of any of claims 1 to 5, wherein portion (I) comprises the amino acid sequence of figure 1A (amino acid sequence of D-TAT: RRRQRRKKRG) or figure 1B (generic amino acid sequence of D-TAT: Xn- RRRQRRKKR-Xn).

7. The fusion protein of any of claims 1 to 6, wherein portion (II) comprises a partial or full-length BH3-domain sequence which induces apoptosis.

8. The fusion protein of any of claims 1 to 7, wherein portion (II) comprises a partial or full-length BH3-domain sequence which interacts with at least one Bcl-2 family protein.

9. The fusion protein of any of claims 1 to 8, wherein portion (II) comprises a partial or full-length BH3-domain sequence which activates or sensitizes at least one pro-apoptotic member of the Bcl-2 family.

10. The fusion protein of any of claims 1 to 9, wherein portion (II) comprises a partial or full-length BH3-domain sequence from a BH3-only protein selected from the group consisting of Bid, Bad, Noxa, Puma, Bim and Bik.

11. The fusion protein of any of claims 1 to 10, wherein portion (II) comprises a partial or full-length BH3-domain sequence in its native form composed of L-amino acids or in its inverted form composed of D-amino acids.

12. The fusion protein of any of claims 1 to 11, wherein portion (II) comprises the amino acid sequence of figures 2A, 2B, 2C, 2D, 2E or 2F (amino acid sequences of Bid, Bad, Noxa, Puma, Bim and Bik) or a fragment thereof comprising a partial or full-length BH3-domain sequence.

13. A pharmaceutical composition comprising a fusion protein of any of claims 1 to 12 and optionally a pharmaceutically acceptable carrier, adjuvant and/or vehicle.

14. Use of a fusion protein of any of claims 1 to 12 for the treatment and/or prophylaxis of diseases caused by defective apoptosis, cancer, e.g., Hodgkin lymphoma, non-Hodgkin lymphoma, histocytic lymphoma, cancers of the brain (glioblastomas), ovarian, genitourinary tract, colon, liver, colorectal tract, pancreas, breast, prostate, lymphatic system, stomach, larynx and lung, including lung adenocarcinoma and small cell lung cancer and/or skin, e.g. melanoma or non-melanoma skin cancer, including basal cell and squamous cell carcinomas as well as psoriasis, Behcet's syndrome, pemphigus vulgaris.

15. Use of a fusion protein any of claims 1 to 12 for the manufacture of a pharmaceutical composition for the treatment and/or prophylaxis of diseases caused by defective apoptosis, cancer, e.g., Hodgkin lymphoma, non-Hodgkin lymphoma, histocytic lymphoma, cancers of the brain (glioblastomas), ovarian, genitourinary tract, colon, liver, colorectal tract, pancreas, breast, prostate, lymphatic system, stomach, larynx and lung, including lung adenocarcinoma and small cell lung cancer and/or skin, e.g. melanoma or non-melanoma skin cancer, including basal cell and squamous cell carcinomas as well as psoriasis, Behcet's syndrome, pemphigus vulgaris.
